# EUROPEAN PATENT APPLICATION

(11) **EP 3 862 051 A1**
(43) Date of publication of application: **11.08.2021**
(21) Application number: 18940309.0
(22) Date of filing: 15.11.2018
(51) Int. Cl.: A61Q 17/04, A61K 8/29, A61K 8/37, A61K 8/34, C09D 129/04

(54) **MACROMOLECULAR ULTRAVIOLET ABSORBENT, PREPARATION METHOD THEREFOR AND APPLICATION THEREOF**

(71) Applicant: Dalian Institute Of Chemical Physics, Chinese Academy Of Sciences, Liaoning 116023 (CN)
(72) Inventor: XING, Jiacheng, Dalian City, Liaoning 116023 (CN); YUAN, Danhua, Dalian City, Liaoning 116023 (CN); XU, Yunpeng, Dalian City, Liaoning 116023 (CN); LIU, Zhongmin, Dalian City, Liaoning 116023 (CN)
(74) Representative: Ter Meer Steinmeister & Partner
(86) International application number: PCT/CN2018/115723
(87) International publication number: WO 2020/097880

(57) **Abstract**

This application discloses a polymer ultraviolet absorbent and preparation method and application thereof. The synthesis process of the polymer ultraviolet absorbent does not require additional organic solvents, and the reaction by-product is high-purity ethanol, which is relatively economical. green, efficient and environmentally friendly. The polymer ultraviolet absorbent has good water solubility, is convenient to use, safe and non-toxic, and has excellent ultraviolet absorption effect.

## Description

### TECHNICAL FIELD

The present application relates to a polymer ultraviolet absorbent, which belongs to the technical field of daily chemical industry.

### BACKGROUND

With the continuous progress of industrialization, the problem of ozone depletion has become more and more serious. The reduction of the ozone layer has led to a significant increase in the ultraviolet radiation on the ground. Excessive ultraviolet light is a serious threat to human health. At the same time, due to long-term exposure to ultraviolet light, many materials will be made in a state of discoloration, brittleness, and performance degradation. Ultraviolet light is an electromagnetic wave with a shorter wavelength and higher energy, and its wavelength range is from 100 to 400nm. Long-wave ultraviolet (UV-A) has a wavelength ranging from 320 to 400nm, and short-wave ultraviolet (UV-B) has wavelength ranging from 280 to 320nm. UV-B is the main cause of sunburn. It has erythema effect on the human body, which can cause the skin to become darken and cause redness and peeling. Ultraviolet absorbents can selectively absorb high-energy ultraviolet light and perform energy conversion to release energy in a manner of heat or harmless low-energy radiation. The current sunscreens mainly use aromatic compounds, such as, benzophenone-3 (i.e., BP3), octyl methoxycinnamate (OMC), and octocrylene. However, many of the aromatic compounds have potential carcinogenic and allergenic risks. Among them, benzophenone-3 is controversial. It has estrogen-like effects and interferes with endocrine. The US FDA stipulates that the highest concentration of benzophenone-3 must not exceed 6%, China stipulates that it cannot exceed 10%, and Sweden has banned the use of this compound. Therefore, the development of a new ultraviolet absorbent has great application prospects.

Polyethylene glycol is non-toxic and has good biocompatibility. Polyethylene glycol has multiple hydrophilic hydroxyl groups, which can relieve skin irritation, improve the moisture retention of the skin surface and effectively improve the comfort of the product. Polyethylene glycol has been approved by the FDA as a pharmaceutical polymer for injectable in vivo. The polymer ultraviolet absorbent formed by using ethylene glycol as the structural unit has excellent water solubility and biological safety. No research report on this type of UV absorbent has been found so far.

The polymerized organosilicon compound is connected by siloxane bonds, and is optically transparent, inert and non-toxic. Due to the diversity nature of polymers, more than 40% of cosmetics and skin care products comprise organosilicon ingredients. The addition of silicon makes skin care products smoother and, at the same time, makes skin more delicate and smoother. The unique water-soluble formula makes the sunscreen, which is difficult to be removed, become safer, not easy to deposit and easy to wash off.

### SUMMARY

According to one aspect of the present application, a polymer ultraviolet absorbent is provided, which has the advantages of good water solubility, convenient use, safety and non-toxicity, and excellent ultraviolet absorption effect.

The polymer ultraviolet absorbent is characterized in that the chemical formula thereof comprises a structural unit as shown in formula I: 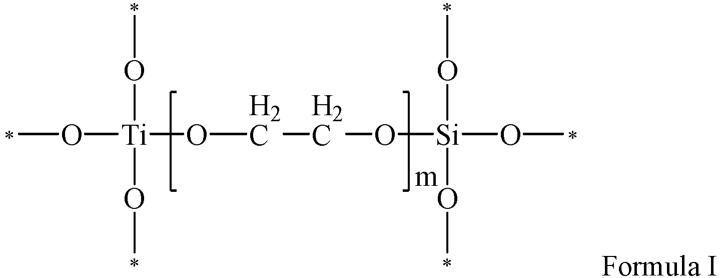 wherein, m=1∼20.

Optionally, a degree of polymerization of the structural unit in the polymer ultraviolet absorbent is in a range from 12 to 20.

Optionally, m=4 in Formula I.

Optionally, m in Formula I depends on the degree of polymerization of polyethylene glycol as raw material.

Optionally, the polymer ultraviolet absorbent is formulated to be an aqueous solution with no less than 0.7 wt%, which has strong absorption of short-wave ultraviolet light.

According to another aspect of the present application, a method for preparing the polymer ultraviolet absorbent is provided, which comprises performing transesterification among a mixture containing polyethylene glycol, titanate and silicate to prepare the polymer ultraviolet absorbent.

Optionally, the titanate is at least one of compounds having a chemical formula shown in formula II: 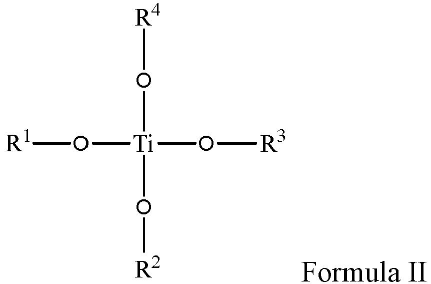 wherein, R¹, R², R³ and R⁴ are independently selected from C₁∼C₈ alkyl group.

Optionally, the titanate comprises at least one of tetraethyl titanate, tetrabutyl titanate, tetraisopropyl titanate, tetrahexyl titanate, and tetraisooctyl titanate.

Optionally, the silicate is at least one of compounds having a chemical formula shown in formula III: 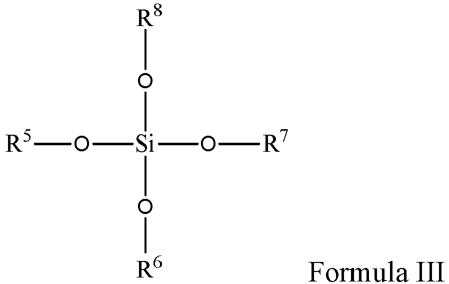 wherein, R⁵, R⁶, R⁷ and R⁸ are independently selected from C₁∼C₄ alkyl group.

Optionally, the silicate comprises at least one of tetramethoxysilane, tetraethyl orthosilicate, tetrapropyl silicate and tetrabutyl silicate.

Optionally, the polyethylene glycol can be one or a mixture of any of polyethylene glycol 200, polyethylene glycol 400, polyethylene glycol 600, and polyethylene glycol 800.

Optionally, the polyethylene glycol comprises at least one of polyethylene glycol 200, polyethylene glycol 400, polyethylene glycol 600, and polyethylene glycol 800.

Optionally, the molar ratio of the polyethylene glycol, titanate and silicate satisfies:
(titanate + silicate): polyethylene glycol = (0.8-1.2) x/4;
titanate: silicate=0.01∼1;
wherein x is the number of moles of hydroxyl groups contained in each mole of polyethylene glycol;
the number of moles of the titanate, silicate, and polyethylene glycol are all based on the number of moles of the substance itself.

Optionally, the upper limit of the molar ratio of (titanate + silicate) to polyethylene glycol is 0.85x/4, 0.9x/4, 0.95x/4, 1.0x/4, 1.1x/4, 1.15x/4 or 1.2x/4, and the lower limit thereof is 0.8x/4, 0.85x/4, 0.9x/4, 0.95x/4, 1.0x/4, 1.1x/4 or 1.15x/4.

Optionally, the upper limit of the molar ratio of the titanate to the silicate is 0.02, 0.05, 0.08, 0.1, 0.2, 0.5, 0.8 or 1, and the lower limit thereof is 0.01, 0.02, 0.05, 0.08, 0.1, 0.2, 0.5 or 0.8.

Optionally, the conditions for the transesterification are: a reaction temperature ranges from 80 to 180 °C, and a reaction time ranges from 2 to 10 hours in an inactive atmosphere.

Optionally, the inactive atmosphere includes at least one of nitrogen and inert gas atmosphere.

Optionally, the transesterification is carried out under stirring condition.

Optionally, the upper limit of the reaction temperature is 90°C, 100°C, 110°C, 120°C, 130°C, 140°C, 150°C, 160°C, 170°C or 180°C, and the lower limit thereof is 80°C, 90°C, 100°C, 110°C, 120°C, 130°C, 140°C, 150°C, 160°C or 170°C.

Optionally, the upper limit of the reaction time is 3 hours, 4 hours, 5 hours, 6 hours, 7 hours, 8 hours, 9 hours or 10 hours, and the lower limit thereof is 2 hours, 3 hours, 4 hours, 5 hours, 6 hours, 7 hours, 8 hours or 9 hours.

Optionally, the reaction time ranges from 2 to 6 hours.

Optionally, the conversion rate of the transesterification ranges from 60% to 80%.

Optionally, the conditions for the transesterification reaction further comprise performing vacuum distillation thereafter.

Optionally, the conditions of the vacuum distillation comprise: a vacuum degree ranges from 0.01 to 5kPa, a vacuum distillation temperature ranges from 170 to 230°C, and a vacuum distillation time ranges from 0.5 to 5 hours.

Optionally, in the vacuum distillation process, the upper limit of the vacuum degree is 0.02kPa, 0.05kPa, 0.1kPa, 0.5kPa, 1kPa, 2kPa, 3kPa, 4kPa or 5kPa, and the lower limit thereof is 0.01kPa, 0.02kPa, 0.05 kPa, 0.1kPa, 0.5kPa, 1kPa, 2kPa, 3kPa or 4kPa.

Optionally, in the vacuum distillation process, the upper limit of the vacuum distillation temperature is 180°C, 190°C, 200°C, 210°C, 220°C or 230°C, and the lower limit thereof is 170°C, 180°C, 190°C, 200°C, 210°C or 220°C. Optionally, in the vacuum distillation process, the upper limit of a vacuum distillation time is 1 hour, 2 hours, 3 hours, 4 hours or 5 hours, and the lower limit thereof is 0.5 hour, 1 hour, 2 hours, 3 hours or 4 hours.

Optionally, the vacuum degree ranges from 1 to 5kPa.

Optionally, the conversion rate of the transesterification is greater than 90%.

Optionally, the method comprises:
a) mixing polyethylene glycol, titanate and silicate, and then performing the transesterification under stirring conditions and in an inactive protection atmosphere, wherein the reaction temperature ranges from 80 to 180°C, and the reaction time ranges from 2 to 10 hours;
b) after the reaction in step a), performing vacuum distillation to prepare the polymer ultraviolet absorbent, during which the vacuum degree ranges from 0.01 to 5kPa, the reaction temperature ranges from 170 to 230°C, and the reaction time ranges from 0.5 to 5 hours.

As a specific embodiment, the method comprises:
1) mixing polyethylene glycol, titanate and silicate uniformly in a three-necked flask, and performing the transesterification under stirring conditions during which a distillation device is connected to the three-necked flask and nitrogen is passed in the three-necked flask for protection, wherein the reaction temperature ranges from 80 to 180 °C, the reaction time ranges from 2 to 10 hours, and the conversion rate of the transesterification ranges from 60% to 80%;
2) after step 1), connecting the distillation device to the water pump or oil pump for vacuum distillation to make the transesterification more complete, wherein the vacuum degree is controlled to range from 0.01 to 5kPa, the reaction temperature ranges from 170 to 230°C, the reaction time ranges from 0.5 to 5 hours, and the conversion rate of the transesterification is greater than 90%.

According to a further aspect of the present application, at least one of the polymer ultraviolet absorbents described in any one of the above and the polymer ultraviolet absorbent prepared according to the method described in any one of the above is used in the fields of cosmetics and textiles.

The polymer ultraviolet absorbent of the present application can also be formulated into other ingredients of cosmetics, such as preservative/antioxidant, water, organic solvent, thickener, softener, emulsifier, defoamer, humectant, fragrance, surfactant, filler, chelating agent, anionic polymer, cationic polymer, non-ionic polymer or amphoteric polymer and a mixture thereof, propellant; acid-alkalizer, dye, colorant, and is especially suitable for the ingredient of the formula to provide additional UV-B protection.

The ultraviolet absorbent of the present application is also suitable for sun protection of textiles.

The ultraviolet absorbent has excellent hydrophilicity and ultraviolet absorption performance, and is used in sunscreen cosmetics, belonging to the technical field of daily chemical industry.

In the present application, "C₁∼C₈, C₁ to C₄" and the like all refer to the number of carbon atoms contained in the group.

In the present application, "alkyl" is a group formed by the loss of any hydrogen atom on the molecule of an alkane compound.

The beneficial effects that the present application can achieve comprise:
1) The polymer ultraviolet absorbent of the present application has strong absorption in the UV-B band, which can effectively prevent the skin or related materials from reddening and aging under ultraviolet radiation.
2) The synthesis process of the polymer ultraviolet absorbent in the present application does not require additional organic solvents, and the reaction by-product is high-purity ethanol, which is economical, green, efficient, environmentally friendly, safe and non-toxic.

### BRIEF DESCRIPTION OF DRAWINGS

Figure 1 shows the thermogravimetric spectrum of the 6# polymer ultraviolet absorbent in Example 2.
Figure 2 shows the UV absorption spectrum of the 1# polymer UV absorbent in Example 1.
Figure 3 shows the silicon nuclear magnetic resonance spectrum of the 5# polymer ultraviolet absorbent in Example 1.
Figure 4 shows the carbon nuclear magnetic resonance spectrum of the 5# polymer ultraviolet absorbent in Example 1.

### DETAILED DESCRIPTION

The present application will be described in detail below with reference to the examples, but the present application is not limited to these examples.

Unless otherwise specified, the raw materials in the examples of the present application are all commercially available.

The analysis methods in the examples of the present application are as follows.

Thermogravimetric analysis is conducted by TA Q-600 thermogravimetric analyzer produced by TA Instruments.

UV-Visible spectra analysis is conducted by CARY-5000 UV-Visible absorption spectrometer produced by VARIAN.

Silicon nuclear magnetic resonance and carbon nuclear magnetic resonance analysis for the synthesized polymer ultraviolet absorbent are conducted by the Bruker Avance111 solid-state nuclear magnetic resonance instrument produced by Bruker.

The conversion rate of the transesterification in the examples of the present application is calculated as follows.

According to the number of moles n of the by-product alcohols distilled out during the reaction, the number of groups participating in the transesterification is determined to be n, and the total number of moles of titanate and silicate in the reaction raw materials is m, and then the conversion rate of the transesterification is n: 4m.

According to an embodiment of the present application, the polymer ultraviolet absorbent is composed of structural unit represented by the following formula: 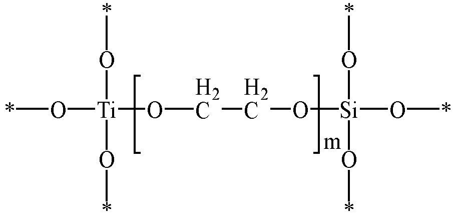 m is 2 or more, depending on the degree of polymerization of polyethylene glycol.

Optionally, it is characterized in that the method comprises the following steps:
a) mixing polyethylene glycol, titanate and silicate uniformly in a three-necked flask, and performing the transesterification under stirring conditions during which a distillation device is connected to the three-necked flask and nitrogen is passed in the three-necked flask for protection, wherein the reaction temperature ranges from 80 to 180 °C, the reaction time ranges from 2 to 10 hours, and the conversion rate of the transesterification ranges from 60% to 80%;
b) after step 1), connecting the distillation device to the water pump or oil pump for vacuum distillation to make the transesterification more complete,
wherein the vacuum degree is controlled to range from 0.01 to 5kPa, the reaction temperature ranges from 170 to 230°C, the reaction time ranges from 0.5 to 5 hours, and the conversion rate of the transesterification is greater than 90%.

Optionally, the formula of titanate and silicate in step a) is M(OR)ₙ, wherein M is Ti or Si, R is an alkyl group, M(OR)ₙ includes one of tetraethyl titanate, tetrabutyl titanate, tetraisopropyl titanate, tetrahexyl titanate, tetraisooctyl titanate, tetramethoxysilane, tetraethyl orthosilicate, tetrapropyl silicate and tetrabutyl silicate.

Optionally, the polyethylene glycol in step a) may be one or a mixture of any of polyethylene glycol 200, polyethylene glycol 400, polyethylene glycol 600, and polyethylene glycol 800.

Optionally, in the step a), silicate, titanate and polyethylene glycol satisfy the following molar ratios: M(OR)ₙ/R-(OH)ₓ = (0.8∼1.2)x/n.

Optionally, the step a) is carried out under nitrogen protection, the reaction temperature thereof ranges from 80 to 180 °C, and the reaction time thereof ranges from 2 to 6 hours.

Optionally, the step b) is carried out under vacuum distillation conditions, and the vacuum degree thereof ranges from 1 to 5kPa.

### Example 1

In Example 1, the specific process is as follows.

80.76g PEG-200, 38.4g tetraethyl orthosilicate and 1.76g tetraethyl titanate are added into a three-necked flask which is connected to a distillation device, and then temperature is heat up to 175°C under stirring and nitrogen protection, and the reaction time is 4 hours. During this process, a large amount of ethanol is distilled out, and the conversion rate of the transesterification is 75%. Then a vacuum device is connected to the distillation device, and the transesterification continues under vacuum distillation conditions, wherein the vacuum degree of the reaction system is controlled to be 1kPa and the temperature is raised to 200 °C. After reacting for 1 hour, the transesterification is stopped. After the temperature is naturally cooled to be room temperature, the resulting sample is taken and labeled as 1# sample ,and the conversion rate of the transesterification is 93%.

### Preparation of 2#sample

The preparation of 2#sample is similar to the preparation of sample #1. The difference of preparation of 2#sample from the preparation of sample #1 is that under nitrogen protection, the temperature is heated up to 180°C and the transesterification is performed for 2 hours.

### Preparation of 3#sample

The preparation of 3#sample is similar to the preparation of sample #1. The difference of preparation of 3#sample from the preparation of sample #1 is that under the nitrogen protection, the temperature is heated up to 80°C and the transesterification is performed for 10 hours.

### Preparation of 4#sample

The preparation of 4# sample is similar to the preparation of sample #1. The difference of preparation of 4#sample from the preparation of sample #1 is that the vacuum degree of the reaction system is controlled to be 0.01kPa, the temperature is raised to 230 °C, and the reaction time of the vacuum distillation process is 0.5 hour.

### Preparation of 5#sample

The preparation of 5# sample is similar to the preparation of sample #1. The difference of preparation of 5#sample from the preparation of sample #1 is that the vacuum degree of the reaction system is controlled to be 0.5kPa, the temperature is raised to 170 °C, and the reaction time of the vacuum distillation process is 5 hours.

During the preparation of 2# to 5# samples, the conversion rate of the transesterification before vacuum distillation process ranges from 60% to 80%, and the conversion rate of the transesterification after vacuum distillation process is greater than 90%.

### Example 2

In Example 2, the specific process is as follows.

80.76g PEG-200, 38.4g tetraethyl orthosilicate and 3.52g tetraethyl titanate are added into a three-necked flask which is connected to a distillation device, and then temperature is heat up to 150°C under stirring and nitrogen protection, and the reaction time is 6 hours. During this process, a large amount of ethanol is distilled out, and the conversion rate of the transesterification is 77%. Then a vacuum device is connected to the distillation device, and the transesterification continues under vacuum distillation conditions, wherein the vacuum degree of the reaction system is controlled to be 2kPa and the temperature is raised to 180 °C. After reacting for 1 hour, the transesterification is stopped. After the temperature is naturally cooled to be room temperature, the resulting sample is taken and labeled as 6# sample ,and the conversion rate of the transesterification is 92%.

### Preparation of 7#sample

The preparation of 7# sample is similar to the preparation of 6#sample. The difference of preparation of 7#sample from the preparation of 6#sample is that PEG-400 replaces PEG-200 in the preparation of 6#sample, and the amount of PEG-400 added is 161.52g; tetramethoxysilane replaces tetraethyl orthosilicate in the preparation of 6#sample, and the amount thereof added is 28.1g; and tetrabutyl titanate replaces tetraethyl titanate in the preparation of 6#sample, and the amount thereof added is 5.2 g.

### Preparation of 8#sample

The preparation of 8# sample is similar to the preparation of 6#sample. The difference of preparation of 8#sample from the preparation of 6#sample is that PEG-600 replaces PEG-200 in the preparation of 6#sample, and the amount of PEG-600 added is 242.3g; tetrapropyl silicate replaces tetraethyl orthosilicate in the preparation of 6#sample, and the amount thereof added is 48.7g; and tetraisopropyl titanate replaces tetraethyl titanate in the preparation of 6#sample, and the amount thereof added is 4.38 g.

### Preparation of 9#sample

The preparation of 9# sample is similar to the preparation of 6#sample. The difference of preparation of 9#sample from the preparation of 6#sample is that PEG-800 replaces PEG-200 in the preparation of 6#sample, and the amount of PEG-800 added is 162g; tetrabutyl silicate replaces tetraethyl orthosilicate in the preparation of 6#sample, and the amount thereof added is 29.5g; and tetrahexyl titanate replaces tetraethyl titanate in the preparation of 6#sample, and the amount thereof added is 6.2 g.

### Preparation of 10#sample

The preparation of 10# sample is similar to the preparation of 6#sample. The difference of preparation of 10#sample from the preparation of 6#sample is that tetraisooctyl titanate replaces tetraethyl titanate in the preparation of 6#sample, and the amount thereof added is 8.3 g.

### Example 3

In Example 3, the specific process is as follows.

80.76g PEG-200, 38.4g tetraethyl orthosilicate and 1.76g tetraethyl titanate are added into a three-necked flask which is connected to a distillation device, and then temperature is heat up to 120°C under stirring and nitrogen protection, and the reaction time is 8 hours. During this process, a large amount of ethanol is distilled out, and the conversion rate of the transesterification is 73%. Then a vacuum device is connected to the distillation device, and the transesterification continues under vacuum distillation conditions, wherein the vacuum degree of the reaction system is controlled to be 1kPa and the temperature is raised to 220 °C. After reacting for 1 hour, the transesterification is stopped. After the temperature is naturally cooled to be room temperature, the resulting sample is taken, and the conversion rate of the transesterification is 96%.

### Example 4 Ultraviolet Absorption Test

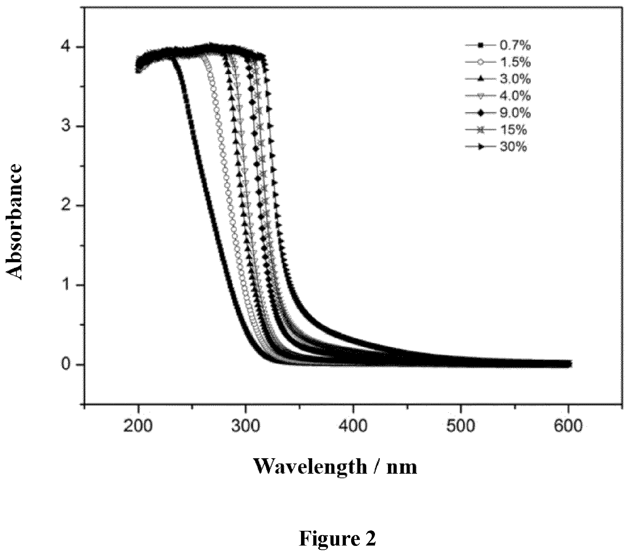

The samples prepared in Example 1 to Example 3 are subjected to an ultraviolet absorption test respectively, typical results of which are shown in Figure 2. Figure 2 corresponds to the ultraviolet absorption spectrum of 1# sample in Example 1.

Water-soluble polymer ultraviolet absorbents are prepared and subjected to ultraviolet absorption test. Different weights of polymer ultraviolet absorbents are dissolved in deionized water, and different weight percentages of polymer ultraviolet absorbent solution (i.e., 0.7wt%, 1.5wt%, 3.0wt%, 4.0wt%, 9.0wt%, 15wt %, 30wt%) are prepared after the polymer ultraviolet absorbents are dissolved completely. Deionized water is used as blank calibration, and scanning is performed in the range from 200 to 600nm with CARY 5000 UV-Visible Spectrophotometer produced by VARIAN. It can be found that, when the concentration of the polymer ultraviolet absorbent is 0.7wt%, the polymer ultraviolet absorbent has a strong absorption capacity for ultraviolet light in the UV-B band. As the amount of the polymer ultraviolet absorbent increases, the ultraviolet absorption capacity is significantly improved.

The test results of other samples are similar to the above.

### Example 5 Thermogravimetric Analysis

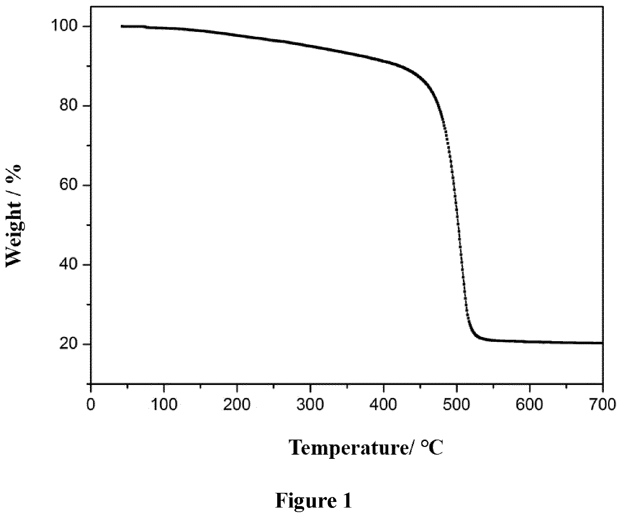

Thermogravimetric analysis is performed on the samples prepared in Example 1 to Example 3, under the condition that a heating rate is 10°C/min, the temperature is heated to 700°C, and the nitrogen flow rate is 100mL/min, the typical result of which is shown in Figure 1. Figure 1 corresponds to the thermogravimetric curve of 6# sample in Example 2.

It can be seen from the figure that 6# sample in Example 2 decomposes at 500°C, and thus the prepared polymer ultraviolet absorbent has a higher thermal decomposition temperature which is 500°C. It also proves that the raw materials are successfully polymerized through the transesterification and the polymer ultraviolet absorbent with good thermal stability is formed.

The test results of other samples are similar to the above.

### Example 6 Nuclear Magnetic Resonance Analysis

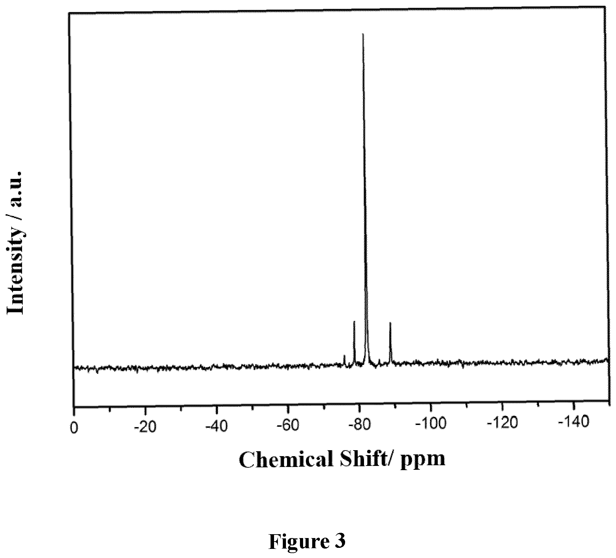

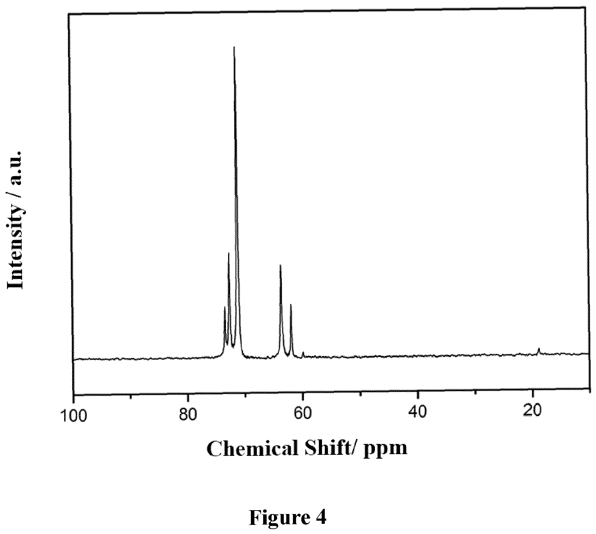

The samples prepared in Example 1 to Example 3 are analyzed by nuclear magnetic resonance, and ¹³C and ²⁹Si nuclear magnetic resonance spectra are used to characterize the samples. Typical spectra are shown in Figures 3 and 4. Figure 3 corresponds to the silicon nuclear magnetic resonance spectra of the 5# polymer ultraviolet absorbent in Example 1. Figure 4 corresponds to the carbon nuclear magnetic resonance spectrum of the 5# polymer ultraviolet absorbent in Example 1.

It can be seen from Figure 3 that the chemical environment around the silicon element in the 5# polymer ultraviolet absorbent in Example 1 is mainly based on the four-coordination of silicon and oxygen. Compared with the chemical shift of silicon in the raw material, it has obvious shift, proving that polymer is formed, and silicon element is linked to polyethylene glycol.

It can be seen from Figure 4 that the chemical environment around the 5# carbon element in the polymer ultraviolet absorbent in Example 1 is mainly based on the carbon in polyethylene glycol, and the nuclear magnetic resonance intensity of the carbon element on the side chain of the alkyl group on the silicate and titanate in the raw materials is significantly reduced, which fully shows that the alkyl side chain in the silicate and titanate in the raw material is broken and removed, which proves that titanium silicon polymer with polyethylene glycol as chain element is formed.

The test results of other samples are similar to the above.

The above examples are only illustrative, and do not limit the present application in any form. Any change or modification, made by the skilled in the art based on the technical content disclosed above, without departing from the spirit of the present application, is equivalent example and falls within the scope of the present application.

## Claims

1. A polymer ultraviolet absorbent, wherein a chemical formula of the polymer ultraviolet absorbent comprises a structural unit as shown in formula I: 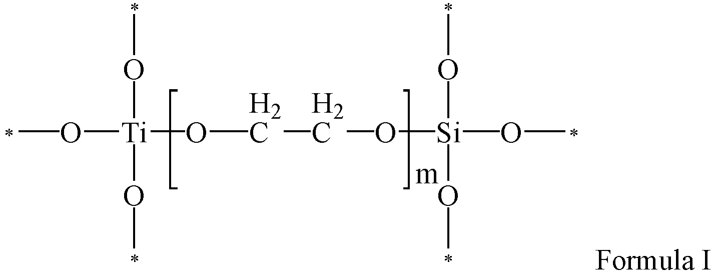 wherein, m=1∼20.

2. A method for preparing the polymer ultraviolet absorbent according to claim 1 comprising performing transesterification among a mixture containing polyethylene glycol, titanate and silicate to prepare the polymer ultraviolet absorbent.

3. The method according to claim 2, wherein the titanate is at least one of compounds having a chemical formula shown in formula II, 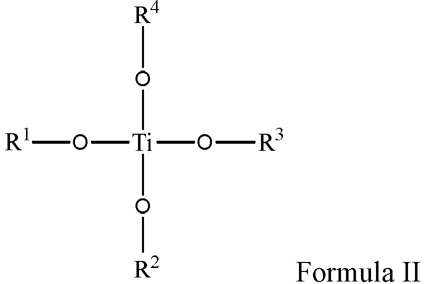 wherein R¹, R², R³ and R⁴ are independently selected from C₁∼C₈ alkyl group.

4. The method according to claim 3, wherein the titanate comprises at least one of tetraethyl titanate, tetrabutyl titanate, tetraisopropyl titanate, tetrahexyl titanate and tetraisooctyl titanate.

5. The method according to claim 2, wherein the silicate is at least one of compounds having a chemical formula shown in formula III: 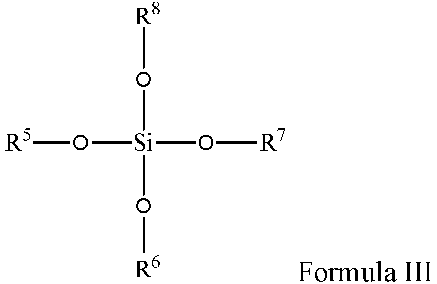 wherein, R⁵, R⁶, R⁷ and R⁸ are independently selected from C₁∼C₄ alkyl group.

6. The method according to claim 5, wherein the silicate comprises at least one of tetramethoxysilane, tetraethyl orthosilicate, tetrapropyl silicate and tetrabutyl silicate.

7. The method according to claim 2, wherein a molar ratio of polyethylene glycol, titanate and silicate satisfies:
(titanate + silicate): polyethylene glycol = (0.8-1.2) x/4;
titanate: silicate=0.01∼1;
wherein x is the number of moles of hydroxyl groups contained in each mole of polyethylene glycol;
the number of moles of the titanate, silicate, and polyethylene glycol are all based on the number of moles of the substance itself.

8. The method according to claim 2, wherein conditions for the transesterification are: a reaction temperature ranges from 80 to 180 °C, and a reaction time ranges from 2 to 10 hours in an inactive atmosphere.

9. The method according to claim 8, wherein the reaction time ranges from 2 to 6 hours.

10. The method according to claim 8, wherein the conditions for the transesterification reaction further comprise performing vacuum distillation thereafter.

11. The method according to claim 10, wherein conditions of the vacuum distillation comprise: a vacuum degree ranges from 0.01 to 5kPa, a vacuum distillation temperature ranges from 70 to 230°C, and a vacuum distillation time ranges from 0.5 to 5 hours.

12. The method according to claim 11, wherein the vacuum degree ranges from 1 to 5kPa.

13. The method according to claim 2, wherein the method comprises following steps:
a) mixing polyethylene glycol, titanate and silicate, and then performing the transesterification under stirring conditions and in an inactive protection atmosphere, wherein the reaction temperature ranges from 80 to 180°C, and the reaction time ranges from 2 to 10 hours;
b) after the reaction in step a), performing vacuum distillation to prepare the polymer ultraviolet absorbent, during which a vacuum degree ranges from 0.01 to 5kPa, a reaction temperature ranges from 170 to 230 °C, and a reaction time ranges from 0.5 to 5 hours.

14. The use of the polymer ultraviolet absorbent according to claim 1 or the polymer ultraviolet absorbent prepared according to the method of any one of claims 2 to 13 in the fields of cosmetics and textiles.
